Europäisches Patentamt

European Patent Office

Office européen des brevets

(19)

(11) Numéro de publication : **0 300 842 B1**

(12) **FASCICULE DE BREVET EUROPEEN**

(45) Date de publication du fascicule du brevet :
**17.04.91 Bulletin 91/16**

(51) Int. Cl.⁵ : **A61K 31/35, A61K 7/48, A61K 7/00, A61K 9/50**

(21) Numéro de dépôt : **88401421.8**

(22) Date de dépôt : **10.06.88**

(54) Composition pharmaceutique ou cosmétique contenant de l'hydroquinone et de l'acide kojique en liposomes.

(30) Priorité : **12.06.87 FR 8708236**

(43) Date de publication de la demande :
**25.01.89 Bulletin 89/04**

(45) Mention de la délivrance du brevet :
**17.04.91 Bulletin 91/16**

(84) Etats contractants désignés :
**AT BE CH DE ES FR GB GR IT LI LU NL SE**

(56) Documents cités :
**EP-A- 0 208 799**
**WO-A-85/04101**
**FR-A- 2 540 381**
**GB-A- 1 303 566**
**GB-A- 1 319 455**

(56) Documents cités :
**GB-A- 2 052 973**
**US-A- 4 545 982**
**CHEMICAL ABSTRACTS, vol. 102, no. 2, 14 janvier 1985, page 348, résumé no. 12207k, Columbus, Ohio, US; & JP-A-59 157 009 (YAKU-RIGAKU CHUO KENKYUSHO K.K.) 06-09-1984**

(73) Titulaire : **LVMH RECHERCHE**
**48-50, rue de Seine**
**F-92703 Colombes Cédex (FR)**

(72) Inventeur : **Meybeck, Alain**
**les Poissons-apt 242 20 ter rue de Bezons**
**F-92400 Courbevoie (FR)**

(74) Mandataire : **Portal, Gérard et al**
**Cabinet Beau de Loménie 55, rue d'Amsterdam**
**F-75008 Paris (FR)**

## Description

La présente invention concerne essentiellement une composition de principes actifs pour la préparation d'une composition pharmaceutique ou cosmétique contenant de l'hydroquinone et de l'acide kojique en liposomes et une composition pharmaceutique, notamment dermatologique, à activité dépigmentante ou anti-inflammatoire, ou cosmétique comprenant de l'acide kojique et de l'hydroquinone en liposomes.

Comme composition ayant une activité dépigmentante importante, on peut citer le "Trio de Kligman" qui est basé sur une combinaison de vitamine A acide ou trétinoïne, d'hydroquinone et d'un stéroïde constitué par la déxaméthasone. Cependant, l'utilisation d'une telle composition a été en pratique limitée en raison d'un caractère irritant élevé, causé par la présence de la vitamine A acide qui est bien connue par son caractère irritant, et des effets indésirables dus à l'hypervitaminose A (voir The Journal of Investigative Dermatology, volume 73, N°5, partie I, pages 354-358, et en particulier l'avant-dernier paragraphe de la page 357, publié en 1979).

Par ailleurs, on connaît déjà l'emploi de phases lamellaires lipidiques hydratées ou de liposomes dans des compositions pharmaceutiques ou des compositions cosmétiques dans lesquelles on incorpore divers principes actifs (FR-A-2 540 381).

Il a maintenant été découvert, de manière tout à fait surprenante et inattendue qu'une combinaison d'acide kojique ou ses dérivés, notamment ses sels ou esters, et d'hydroquinone, dont au moins l'un est incorporé dans des phases lamellaires lipidiques hydratées ou dans des liposomes, permet de préparer une composition pharmaceutique, notamment dermatologique, à activité dépigmentante ou anti-inflammatoire ; ou cosmétique particulièrement avantageuse. Sans doute, cette activité est due à un effet de synergie.

Un tel effet de synergie est en outre obtenu de manière particulièrement améliorée si l'on incorpore au moins en partie une combinaison d'acide kojique ou ses dérivés, notamment ses sels ou esters, et d'hydroquinone dans des phases lamellaires lipidiques hydratées ou dans des liposomes.

Ainsi, la présente invention a pour but de résoudre le nouveau problème technique consistant en la fourniture d'une nouvelle formulation contenant de l'hydroquinone permettant de préparer des compositions pharmaceutiques, notamment dermatologiques, à activité dépigmentante ou anti-inflammatoire, ou des compositions cosmétiques à efficacité accrue.

La présente invention résout pour la première fois de manière satisfaisante ce nouveau problème technique d'une manière particulièrement simple, très aisée à mettre en oeuvre à l'échelon industriel.

Ainsi, selon un premier aspect, la présente invention fournit une composition de principes actifs pour la préparation d'une composition pharmaceutique ou cosmétique contenant de l'hydroquinone, caractérisée en ce qu'elle contient en outre de l'acide kojique ou ses dérivés, notamment ses sels ou esters ; au moins l'un parmi l'hydroquinone et l'acide kojique ou ses dérivés, notamment ses sels ou esters, étant sous forme au moins en partie incorporée dans des phases lamellaires lipidiques hydratées ou dans des liposomes.

Selon un deuxième aspect, la présente invention concerne aussi une composition pharmaceutique, notamment dermatologique, à activité dépigmentante ou anti-inflammatoire ; ou une composition cosmétique, caractérisée en ce qu'elle comprend de l'acide kojique ou ses dérivés, notamment ses sels ou esters et de l'hydroquinone ; au moins l'un parmi l'hydroquinone et l'acide kojique ou ses dérivés, notamment ses sels ou esters, étant sous forme ou moins en partie incorporée dans des phases lamellaires lipidiques hydratées ou dans des liposomes.

De préférence, l'hydroquinone et l'acide kojique ou ses dérivés, notamment ses sels ou esters, sont au moins en partie incorporés dans des phases lamellaires lipidiques hydratées ou dans des liposomes.

De préférence, l'acide kojique ou ses dérivés, notamment ses sels ou esters, constitue de 0,5 à 4%, encore de préférence 0,5 à 2%, et encore mieux 1% en poids par rapport au poids total de la composition.

Selon une autre caractéristique de l'invention, l'hydroquinone constitue de préférence 0,5 à 6%, de préférence 0,5 à 4%, et encore mieux 2% en poids par rapport au poids total de la composition.

L'invention concerne aussi des procédés de préparation des compositions précitées, caractérisés en ce qu'on incorpore à titre de principes actifs l'hydroquinone en mélange avec l'acide kojique ou ses dérivés, notamment ses sels ou esters, et au moins l'un parmi l'hydroquinone et l'acide kojique ou ses dérivés, notamment ses sels ou esters est au moins en partie incorporé dans des phases lamellaires lipidiques hydratées ou dans des liposomes.

Dans la présente description et les revendications, le terme "lipidique" dans l'expression "phase lamellaire lipidique" couvre toutes les substances comprenant une chaîne carbonée dite grasse, généralement supérieure à 5 atomes de carbone.

Selon l'invention, on utilise des lipides amphiphiles, c'est-à-dire constitués de molécules possédant un groupe hydrophyle indifféremment ionique ou non ionique et un groupe lipophile, ces lipides amphiphiles étant susceptibles de former des phases lamellaires lipidiques en présence d'une phase aqueuse. En particulier, citons parmi ces lipides : les phospholipides, les phosphoaminolipides, les glycolipides, les alcools gras polyoxyéthylénés, les esters de polyols éventuellement polyoxyéthylénés. De telles substances sont par exemple constituées par une lécithine d'oeuf ou de soja, une phosphatidylsérine, une sphyngomyéline, un cérébroside ou un stéarate de polyglycérol oxyéthyléné.

D'autres buts, caractéristiques et avantages apparaîtront clairement à la lumière de la description explicative, qui va suivre, faite en référence à plusieurs exemples illustratifs. Dans les exemples, les pourcentages sont donnés en poids sauf indication contraire.

## EXEMPLE 1 SELON L'INVENTION

Préparation d'une composition de principes actifs contenant de l'hydroquinone et de l'acide kojique dans des liposomes (dite composition I1).

On prend 1 g d'acide kojique et 2 g d'hydroquinone que l'on dissout dans 30 ml de dichlorométhane auxquels on ajoute 2 g de lécithine de soja hydrogénée, éventuellement en présence d'un antioxydant lipophile, par exemple 0,06 g d'alpha-tocophérol.

La solution obtenue peut être traitée pour obtenir une suspension de liposomes par le procédé d'évaporation en récipient rotatif bien connu et qui consiste à déposer une couche lipidique dans le ballon de l'évaporateur rotatif par évaporation du solvant, puis ajouter de l'eau ou une solution aqueuse appropriée.

On peut également utiliser la technique dite de la phase inverse décrite dans le document FR-A-2 399 242.

Cependant, on préfère atomiser la solution obtenue à 65°C, comme décrit dans le document FR-A-2 521 565 de manière à produire une fine poudre que l'on disperse dans 200 ml d'une solution aqueuse tamponnée à pH 7,5 environ. Généralement, on utilise une solution contenant 0,8% en NaCl et 1,5% $NaH_2PO_4$ dite "tampon phosphate", additionnée d'un stabilisateur antioxydant tel que de l'acide ascorbique à la concentration de 0,05%.

On obtient ainsi, après homogénéisation soit aux ultrasons, soit dans un homogénéisateur sous pression, par exemple selon le procédé décrit dans le document FR-A-2 534 487, ladite suspension de liposomes dans laquelle la concentration en acide kojique est de 1% et 2% en hydroquinone et de 1% en lécithine.

Si par exemple, on réalise une homogénéisation par un traitement aux ultrasons pendant 10 min, on obtient une dimension de liposomes moyenne de l'ordre de 112 nm.

On observera qu'on peut naturellement réaliser diverses dilutions en modifiant la quantité d'acide kojique ou d'hydroquinone ajoutée au départ ou en augmentant le volume de la solution de dispersion, ce qui constitue un procédé aisé de préparation de diverses concentrations en substance(s) active(s).

Cette suspension est ensuite transformée en un gel par exemple par addition de 1% d'un agent formateur de gel, comme un polymère vinylique en particulier commercialisé sous la dénomination commerciale Carbopol® 940.

## EXEMPLE 2 COMPARATIF

Préparation d'une composition de principes actifs contenant de l'hydroquinone et de l'acide kojique (dite composition A1).

On dissout 1 g d'acide kojique dans 14 g d'eau distillée, par chauffage à 40°C pendant 10 minutes sous agitation.

On dissout d'autre part, 2 g d'hydroquinone dans une solution contenant 0,5 g d'acide ascorbique et 0,4 g de sulfite de sodium dans 29 g d'éthanol à 95°C.

On mélange la solution aqueuse d'acide kojique et la solution alcoolique d'hydroquinone.

A ce mélange, on ajoute 53,1 g de gel polymère carboxyvinylique (Carbopol® 940).

Pour préparer ce gel de façon classique, on a dispersé 5 g de Carbopol® 940 dans 100 g d'eau en présence de conservateurs et d'un agent chelatant, puis, après gonflement, on neutralise à pH 7,5 avec de la triéthanolamine.

On obtient ainsi une composition gélifiée (I1) contenant en poids 2% d'hydroquinone et 1% d'acide kojique.

## EXEMPLE 3 COMPARATIF (composition dite A2)

Composition de liposomes contenant de l'acide kojique

On procède comme à l'exemple 1, si ce n'est que l'on ajoute seulement 1 g d'acide kojique et pas d'hydroquinone.

## EXEMPLE 4 COMPARATIF (composition dite A3)

Composition de liposomes contenant de l'hydroquinone

On procède comme à l'exemple 1, si ce n'est que l'on ajoute 2 g d'hydroquinone sans acide kojique.

## EXEMPLE 5 COMPARATIF (composition dite A4)

Composition contenant de l'hydroquinone

On prépare une composition comparative sous forme de gel avec le même gel qu'à l'exemple 2 en y incorporant seulement 2% en poids d'hydroquinone.

## EXEMPLE 6 SELON L'INVENTION

Préparation d'une composition liposomale contenant de l'hydroquinone et de l'acide kojique dans la phase aqueuse.

On prépare une solution aqueuse tamponnée de composition suivante :

- acide kojique      1   g
- hydroquinone      2   g
- acide ascorbique    0,5 g
- sulfite de sodium    0,4 g
- tampon phosphate
  pH 7,5 (exemple 2)    44,1 g

Les constituants sont soumis à une agitation à température ordinaire jusqu'à complète solubilisation.

On disperse ensuite dans ladite solution 2 g de poudre de lécithine hydrogénée atomisée selon la méthode mentionnée à l'exemple 1.

Le tout est soumis à une agitation pendant 15 minutes à température ambiante. On obtient ainsi une suspension de liposomes encapsulant, en partie, l'hydroquinone et l'acide kojique.

Cette suspension est homogénéisée par ultra-sons à la puissance de 100 W pendant 10 minutes.

On ajoute enfin 50 g d'un gel de Carbopol® 940 préparé selon les indications de l'exemple 2.

## EXEMPLE 7 SELON L'INVENTION

On procède comme à l'exemple 3 pour incorporer l'acide kojique dans des liposomes.

Ensuite, on ajoute à la composition liposomale obtenue 2 g d'hydroquinone en solution aqueuse tamponnée à pH 7,5 contenant un anti-oxydant tel que l'acide ascorbique à une concentration de 0,05%, de manière à ce que le volume total soit de 200 ml.

Si on le souhaite, on transforme ensuite en gel comme décrit à l'exemple 1.

## EXEMPLE 8 SELON L'INVENTION

On procède comme à l'exemple 7 et de manière inversée entre l'hydroquinone et l'acide kojique en maintenant les quantités de principe actif inchangées.

## EXEMPLE 9

Utilisation des compositions conformes aux exemples 1 à 5 pour constituer une composition pharmaceutique ou cosmétique.

On vérifie l'activité de la composition selon l'invention de l'exemple 1 utilisée comme composition pharmaceutique, notamment dermatologique, ou cosmétique par rapport aux compositions des exemples 2 à 5 en effectuant les expérimentations in vivo suivantes.

Mise évidence de l'activité dépigmentante

Pour étudier l'activité dépigmentante des compositions selon l'invention, on utilise soit des souris Hairless à queue pigmentée, obtenues à 90% dans la portée résultant de deux croisements successifs dont le premier croisement est réalisé à partir d'une souris C 57 noire femelle avec une souris mâle BL6-HRO ou eb-HRO, disponibles au centre de sélection et d'élevage d'animaux de laboratoire, en abrégé CSEAL du CNRS d'Orléans dont les mâles de la portée sont ensuite croisés à nouveau avec une souris femelle C57 noire.

On peut également utiliser des souris Hairless à oreilles pigmentées et yeux noirs Skh : HR-2 disponibles auprès du Temple University et Health Sciences Center Central Animal Facility de Philadelphie.

Pour réaliser les expérimentations, on applique le produit sous forme de gel obtenu aux exemples 1 à 5, 5 jours par semaine pendant 6 semaines.

Ensuite, on prélève par biopsie des fragments d'épiderme de queue ou d'oreilles dans les zones où le produit a été appliqué que l'on met dans du NaBr pendant 2 heures.

On dispose des échantillons d'épiderme entre deux lames de quartz. On effectue un séchage puis une pesée.

Ensuite, on soumet ce fragment d'épiderme séché et pesé à une digestion par la trypsine à 37°C pendant 48 heures.

On effectue ensuite une filtration, une centrifugation à 3000 tr/min pendant 30 minutes.

On récupère le culot mélanique que l'on met en suspension dans l'eau distillée.

On mesure la densité optique de manière classique à 400 nm.

Les valeurs relevées sont ensuite ramenées à 100 mg de poids d'épiderme prélevé soient :

– D.O.T. la valeur moyenne obtenue pour un lot témoin non traité,

– D.O.K. la valeur moyenne obtenue pour un lot traité avec le "Trio de Kligman" considéré comme témoin positif,

– D.O.A. la valeur moyenne obtenue pour le lot traité avec le produit actif soit selon l'invention (I1) soit de comparaison (A1, A2, A3, A4 ou A5 = "Trio de Kligman").

on calcule le pourcentage d'activité par rapport au "Trio de Kligman" de la manière suivante :

$$\text{activité} = \frac{\text{D.O.T.} - \text{D.O.A.}}{\text{D.O.T.} - \text{D.O.K.}} \times 100$$

Les valeurs obtenues avec chacune des compositions des exemples 1 à 5 sont répertoriées au tableau I ci-après. On constate que :

– même sous forme de liposome, l'acide kojique à 1% a une activité presque nulle (A2),

– l'hydroquinone à 2% a également une activité presque nulle sous forme d'un gel classique (A4),

– la combinaison hydroquinone 2% acide kojique 1% en gel classique a une activité peu significative (A1),

– la combinaison (I1) hydroquinone 2% acide kojique 1% en liposomes a une activité importante inattendue et donc très intéressante puisqu'elle atteint presque le tiers de celle du "Trio de Kligman".

Par ailleurs, les compositions selon l'invention ne présentent pas d'effets secondaires et surtout ne présentent pas l'effet irritant de la vitamine A acide qui a toujours constitué une limitation importante à l'utilisation du "Trio de Kligman" basé sur la combinaison de 0,05% de vitamine A acide + dexaméthasone comme stéroïde anti-inflammatoire + 5% d'hydroquinone (composition comparative A5).

On donne ci-après divers exemples de compositions dermatologiques et dermo-cosmétiques.

EXEMPLE 10 SELON L'INVENTION

Préparation cosmétique sous forme liposomique pour atténuer les taches de pigmentation.

| | |
|---|---|
| • hydroquinone | 2 |
| • acide kojique | 1 |
| • lécithine | 1,8 |
| • sitostérol | 0,2 |
| • bisulfite de sodium | 0,5 |
| • gel à 2,5% de Carbopol® 940 | |

| | |
|---|---|
| stabilisé | 30 |
| ● eau q.s.p. | 100 |

On procède selon l'exemple 6, 7 ou 8 le sitostérol étant dissous, avant atomisation, avec la lécithine dans du dichlorométhane.

La suspension de liposomes ainsi obtenue doit être protégée de la lumière et de la chaleur.

## T A B L E A U   I

Résultats d'essais d'activité dépigmentante.

| Composition | I1 Liposomes HQ + A.K. | A1 HQ + A.K. | A2 Liposomes A.K. | A3 Liposomes H.Q. | A4 H.Q. | A5 "Trio de Kligman" |
|---|---|---|---|---|---|---|
| % d'activité par rapport au "Trio de Kligman" | 29 | 13 | 4 | 15 | 3 | 100 |

H.Q. = Hydroquinone
A.K. = Acide Kojique

## Revendications

### Revendications pour les Etats contractants : AT, BE CH DE FR GB IT LI LU NL SE

1. Composition de principes actifs pour la préparation d'une composition pharmaceutique ou cosmétique contenant de l'hydroquinone, caractérisée en ce qu'elle contient en outre de l'acide kojique ou ses dérivés, notamment ses sels ou esters ; au moins l'un parmi l'hydroquinone et l'acide kojique ou ses dérivés, notamment ses sels ou esters, étant sous forme au moins en partie incorporée dans des phases lamellaires lipidiques hydratées ou dans des liposomes.

2. Composition pharmaceutique, notamment dermatologique, à activité dépigmentante ou anti-inflammatoire ; ou cosmétique, contenant de l'hydroquinone, caractérisée en ce qu'elle comprend en outre de l'acide kojique ou ses dérivés, notamment ses sels ou esters ; au moins l'un parmi l'hydroquinone et l'acide kojique ou ses dérivés, notamment ses sels ou esters, étant sous forme au moins en partie incorporée dans des phases lamellaires lipidiques hydratées ou dans des liposomes.

3. Composition selon la revendication 2, caractérisée en ce que l'hydroquinone et l'acide kojique ou ses dérivés, notamment ses sels ou esters, sont au moins en partie incorporés dans des phases lamellaires lipidiques hydratées ou dans des liposomes.

4. Composition pharmaceutique selon la revendication 2 ou 3, caractérisée en ce que l'acide kojique ou ses dérivés, notamment ses sels ou esters, constitue de 0,5 à 4%, de préférence 0,5 à 2%, encore de préférence environ 1% en poids par rapport au poids total de la composition.

5. Composition selon l'une des revendications 2 à 4, caractérisée en ce que l'hydroquinone constitue de 0,5 à 6%, de préférence 0,5 à 4%, encore de préférence environ 2% en poids par rapport au poids total de la composition.

### Revendications pour les Etats contractants suivants : ES, GR

1. Procédé de préparation d'une composition de principes actifs pour la préparation d'une composition pharmaceutique ou cosmétique, caractérisé en ce qu'on incorpore au moins l'un parmi l'acide kojique avec ses dérivés, notamment ses sels ou esters, et l'hydroquinone, au moins en partie dans des phases lamellaires lipidiques hydratées ou dans des liposomes.

2. Procédé de préparation d'une composition pharmaceutique, notamment dermatologique à activité anti-dépigmentante ou anti-inflammatoire ; ou cosmétique, caractérisé en ce qu'on incorpore au moins l'un parmi l'acide kojique avec ses dérivés, notamment ses sels ou esters, et l'hydroquinone, au moins en partie dans des phases lamellaires lipidiques hydratées ou dans des liposomes.

3. Procédé de préparation selon la revendication 2, caractérisé en ce qu'on incorpore en mélange l'acide kojique avec ses dérivés, notamment ses sels ou esters, et l'hydroquinone, au moins en partie dans des phases lamellaires lipidiques hydratées ou dans des liposomes.

4. Procédé selon la revendication 2 ou 3, caractérisé en ce qu'on incorpore de 0,5 à 4%, de préférence 0,5 à 2%, encore de préférence environ 1% en poids d'acide kojique ou ses dérivés, notamment ses sels ou esters, par rapport au poids total de la composition.

5. Procédé selon la revendication 2, 3 ou 4, caractérisé en ce qu'on incorpore l'hydroquinone à raison de 0,5 à 6% de préférence 0,5 à 4%, encore de préférence environ 2% en poids par rapport au poids total de la composition.

## Ansprüche

### Patentansprüche für die Vertragsstaaten AT, BE, CH, DE, FR, GB, IT, LI, LU, NL, SE :

1. Wirkstoffkomposition zur Herstellung einer pharmazeutischen oder kosmetischen Hydrochinon enthaltenden Zusammensetzung, dadurch gekennzeichnet, daß sie weiters Kojisäure oder ihre Derivate, insbesondere ihre Salze oder Ester, enthält, wobei mindestens eines von Hydrochinon und Kojisäure oder ihren Derivaten, insbesondere ihren Salzen oder Estern, zumindest teilweise in in hydrierten lamellaren Lipidphasen oder in Liposomen eingebauter Form vorliegen.

2. Pharmazeutische Hydrochinon enthaltende Zusammensetzung, insbesondere dermatologische Zusammensetzung mit depigmentierender oder entzündungshemmender Aktivität oder kosmetische Zusammensetzung, dadurch gekennzeichnet, daß sie weiters Kojisäure oder ihre Derivate, insbesondere ihre Salze oder Ester enthält, wobei mindestens eines von Hydrochinon und Kojisäure oder ihren Derivaten, insbesondere ihren Salzen oder Estern, zumindest teilweise in in hydrierten lamellaren Lipidphasen oder in Liposomen eingebauter Form vorliegt.

3. Zusammensetzung nach Anspruch 2, dadurch gekennzeichnet, daß das Hydrochinon und die Kojisäure oder ihre Derivate, insbesondere ihre Salze oder Ester, zumindest teilweise in hydrierten lamellaren Lipidphasen oder in Liposomen eingebaut sind.

4. Pharmazeutische Zusammensetzung nach Anspruch 2 oder 3, dadurch gekennzeichnet, daß die Kojisäure oder ihre Derivate, insbesondere ihre Salze oder Ester, 0,5 bis 4 Gew.%, vorzugsweise 0,5 bis 2 Gew.%, noch bevorzugter etwa 1 Gew.%, bezogen auf das Gesamtgewicht der Zusammensetzung, ausmacht.

5. Zusammensetzung nach einem der Ansprüche 2 bis 4, dadurch gekennzeichnet, daß das Hydrochinon 0,5 bis 6 Gew.%, vorzugsweise 0,5 bis 4 Gew.%, noch bevorzugter etwa 2 Gew.%, bezogen auf das Gesamtgewicht der Zusammensetzung, ausmacht.

**Patentansprüche für die Vertragsstaaten ES, GR :**

1. Verfahren zur Herstellung einer Wirkstoffkomposition für die Herstellung einer pharmazeutischen oder kosmetischen Zusammensetzung, dadurch gekennzeichnet, daß man mindestens eines von Kojisäure mit ihren Derivaten, insbesondere ihren Salzen oder Estern, und Hydrochinon zumindest teilweise in hydrierte lamellare Lipidphasen oder in Liposome einbaut.

2. Verfahren zur Herstellung einer pharmazeutischen Zusammensetzung, insbesondere einer dermatologischen Zusammensetzung mit depigmentierender oder entzündungshemmender Aktivität oder einer kosmetischen Zusammensetzung, dadurch gekennzeichnet, daß man mindestens eines von Kojisäure mit ihren Derivaten, insbesondere ihren Salzen oder Estern, und Hydrochinon zumindest teilweise in hydrierte lamellare Lipidphasen oder in Liposome einbaut.

3. Herstellungsverfahren nach Anspruch 2, dadurch gekennzeichnet, daß man die Kojisäure mit ihren Derivaten, insbesondere ihren Salzen oder Estern, und Hydrochinon in Mischung zumindest teilweise in hydrierte lamellare Lipidphasen oder in Liposome einbaut.

4. Verfahren nach Anspruch 2 oder 3, dadurch gekennzeichnet, daß man 0,5 bis 4 Gew.%, vorzugsweise 0,5 bis 2 Gew.%, noch bevorzugter etwa 1 Gew.% Kojisäure oder ihre Derivate, insbesondere ihre Salze oder Ester, bezogen auf das Gesamtgewicht der Zusammensetzung, einbaut.

5. Verfahren nach Anspruch 2, 3 oder 4, dadurch gekennzeichnet, daß man Hydrochinon in einer Menge von 0,5 bis 6 Gew.%, vorzugsweise 0,5 bis 4 Gew.%, noch bevorzugter etwa 2 Gew.%, bezogen auf das Gesamtgewicht der Zusammensetzung, einbaut.

**Claims**

**Claims for the contracting States AT, BE, CH, DE, FR, GB, IT, LI, LU, NL, SE.**

1. A composition of active principles for the preparation of a pharmaceutical or cosmetic composition, containing hydroquinone, characterized in that it also contains kojic acid or derivatives thereof, especially salts or esters thereof ; at least one of the substances hydroquinone and kojic acid or derivatives thereof, especially salts or esters thereof, being in a form which is at least partially incorporated into hydrated lipidic lamellar phases or into liposomes.

2. A pharmaceutical composition, especially a dermatological composition, with skin-lightening or anti-inflammatory activity ; or a cosmetic composition containing hydroquinone, characterized in that it also comprises kojic acid or derivatives thereof, especially salts or esters thereof ; at least one of the substances hydroquinone and kojic acid or derivatives thereof, especially salts or esters thereof, being in a form which is at least partially incorporated into hydrated lipidic lamellar phases or into liposomes.

3. A composition according to claim 2, characterized in that at least part of the hydroquinone and the kojic acid or derivatives thereof, especially salts or esters thereof, is incorporated into hydrated lipidic lamellar phases or into liposomes.

4. A pharmaceutical composition according to claim 2 or 3, characterized in that the kojic acid or derivatives thereof, especially salts or esters thereof, represents from 0.5 to 4%, preferably from 0.5 to 2% and more preferably about 1% by weight, relative to the total weight of the composition.

5. A composition according to any one of claims 2 to 4, characterized in that the hydroquinone represents from 0.5 to 6%, preferably from 0.5 to 4% and more preferably about 2% by weight, relative to the total weight

9

of the composition.

**Claims for the contracting States : ES, GR**

1. A process for the preparation of a composition of active principles for the preparation of a pharmaceutical or cosmetic composition, characterized in that at least one among kojic acid with the derivatives thereof, especially salts or esters thereof ; and hydroquinone are at least partially incorporated into hydrated lipidic lamellar phases or into liposomes.

2. A process for the preparation of a pharmaceutical composition, especially a dermatological composition, with skin-lightening or anti-inflammatory activity ; or a cosmetic composition, characterized in that at least one among kojic acid with the derivatives thereof, especially salts or esters thereof ; and hydroquinone are at least partially incorporated into hydrated lipidic lamellar phases or into liposomes.

3. A process for the preparation according to claim 2, characterized in that a mixture of kojic acid with its derivatives, especially salts or esters thereof, and hydroquinone are incorporated at least partially into hydrated lipidic lamellar phases or into liposomes.

4. A process according to claim 2 or 3, characterized in that the kojic acid or derivatives thereof, especially salts or esters thereof, are incorporated at a percentage of 0.5 to 4%, preferably from 0.5 to 2% and more preferably about 1% by weight, relative to the total weight of the composition.

5. A process according to any one of claims 2, 3 or 4, characterized in that the hydroquinone is incorporated at a rate of 0.5 to 6%, preferably of 0.5 to 4% and more preferably of about 2% by weight, relative to the total weight of the composition.